(19) Europäisches Patentamt
European Patent Office
Office européen des brevets

(11) Veröffentlichungsnummer: **0 028 346**
**B1**

# (12) EUROPÄISCHE PATENTSCHRIFT

(45) Veröffentlichungstag der Patentschrift: **04.08.82**

(51) Int. Cl.³: **C 07 D 249/08**, C 07 D 405/12, A 01 N 43/64

(21) Anmeldenummer: **80106386.8**

(22) Anmeldetag: **20.10.80**

## (54) Trisubstituierte Benzyl-oximether, Verfahren zu ihrer Herstellung sowie ihre Verwendung als Fungizide.

(30) Priorität: **03.11.79 DE 2944446**

(43) Veröffentlichungstag der Anmeldung: **13.05.81 Patentblatt 81/19**

(45) Bekanntmachung des Hinweises auf die Patenterteilung: **04.08.82 Patentblatt 82/31**

(84) Benannte Vertragsstaaten: **AT BE CH DE FR GB IT LI NL SE**

(56) Entgegenhaltungen:
**EP A 0 004 917**
**DE A 2 723 942**
**DE A 2 816 816**

(73) Patentinhaber: **BAYER AG, Zentralbereich Patente, Marken und Lizenzen, D-5090 Leverkusen 1, Bayerwerk (DE)**

(72) Erfinder: **Krämer, Wolfgang, Dr., Am Eckbusch 39/162, D-5600 Wuppertal 1 (DE)**
Erfinder: **Knops, Hans-Joachim, Dr., Claudiusweg 3, D-5600 Wuppertal 1 (DE)**
Erfinder: **Büchel, Karl Heinz, Dr. Prof., Haus an der Dachsdelle Dabringhauser Strasse 42, D-5093 Burscheid (DE)**
Erfinder: **Brandes, Wilhelm, Dr., Eichendorffstrasse 3, D-5653 Leichlingen (DE)**
Erfinder: **Frohberger, Paul-Ernst, Dr., Willi-Baumeister-Strasse 5, D-5090 Leverkusen 1 (DE)**

**EP 0 028 346 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Trisubstituierte Benzyl-oximether, Verfahren zu ihrer Herstellung sowie ihre Verwendung als Fungizide

Die vorliegende Erfindung betrifft neue trisubstituierte Benzyl-oximether, mehrere Verfahren zu ihrer Herstellung sowie ihre Verwendung als Fungizide.

Es ist bereits bekannt geworden, daß bestimmte substituierte Benzyl-oximether, wie insbesondere Dichlorbenzyl-oximether (DE-OS 2 723 942) und Oximino-triazolylethane (EP-A-0 004 917) fungizide Eigenschaften aufweisen. Deren Wirkung ist jedoch in niedrigen Aufwandmengen und -konzentrationen nicht immer befriedigend.

Es wurden neue trisubstituierte Benzyl-oximether der Formel

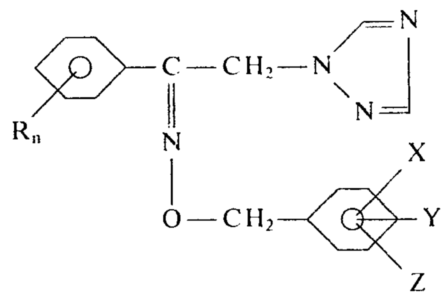

(I)

in welcher

R für Halogen, Alkyl oder Halogenalkyl steht,
n für ganze Zahlen von 1 bis 3 steht,
X, Y, Z gleich oder verschieden sind und für Alkyl, Alkoxy oder Halogen stehen, oder
Y und Z gemeinsam für Methylendioxy stehen,

und deren physiologisch verträglichen Säureadditions-Salze und Metallsalz-Komplexe gefunden.

Die Verbindungen der Formel (I) können in der syn- oder anti-Form vorliegen, vorwiegend fallen sie als Gemische beider Formen an.

Weiterhin wurde gefunden, daß man die trisubstituierten Benzyl-oximether der Formel (I) sowie deren Säureadditions-Salze und Metallsalz-Komplexe erhält, wenn man

a) Oxime der Formel

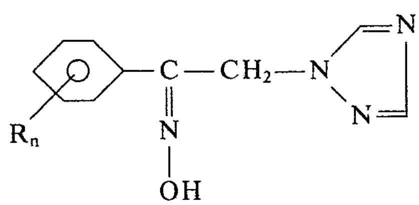

(II)

in welcher
R und n die oben angegebene Bedeutung haben,
mit Benzylhalogeniden der Formel

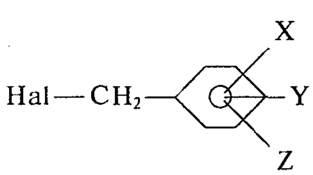

(III)

in welcher
X, Y und Z die oben angegebene Bedeutung haben und
Hal für Chlor oder Brom steht,
gegebenenfalls in Gegenwart einer starken Base und in Gegenwart eines Verdünnungsmittels umsetzt, oder

b) ω-Halogen-acetophenon-oximether der Formel

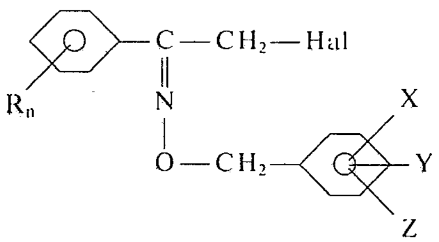

(IV)

in welcher

R, n, X, Y und Z die oben angegebene Bedeutung haben und
Hal für Chlor oder Brom steht,

mit 1,2,4-Triazol in Gegenwart eines Säurebindemittels und in Gegenwart eines Verdünnungsmittels umsetzt, und gegebenenfalls anschließend an die nach dem Verfahren (a) oder (b) erhältlichen Verbindungen eine Säure oder ein Metallsalz addiert.

Die neuen trisubstituierten Benzyl-oximether der Formel (I) weisen starke fungizide Eigenschaften auf. Dabei zeigen überraschenderweise die erfindungsgemäßen Verbindungen eine erheblich höhere Wirkung als die aus dem Stand der Technik bekannten Dichlorbenzyl-oximether, welche chemisch und wirkungsmäßig die naheliegenden Verbindungen sind. Die erfindungsgemäßen Stoffe stellen somit eine Bereicherung der Technik dar.

Die erfindungsgemäßen trisubstituierten Benzyl-oximether sind durch die Formel (I) allgemein definiert. In dieser Formel steht R vorzugsweise für Fluor, Chlor und Brom; für geradkettiges oder verzweigtes Alkyl mit 1 bis 4 Kohlenstoffatomen, wie insbesondere Methyl; sowie für Halogenalkyl mit 1 bis 2 Kohlenstoff- und 1 bis 3 Halogenatomen, wie insbesondere Fluor- und Chloratomen, wobei Trifluormethyl als Beispiel genannt sei. X, Y und Z sind gleich oder verschieden und stehen vorzugsweise für geradkettiges oder verzweigtes Alkyl und Alkoxy mit jeweils 1 bis 4 Kohlenstoffatomen, wie insbesondere Methyl und Methoxy; für Fluor, Chlor und Brom sowie Y und Z gemeinsam auch für Methylendioxy. Der Index n steht vorzugsweise für die Zahlen 1 oder 2.

Besonders bevorzugt sind diejenigen trisubstituierten Benzyl-oximether der Formel (I), in denen X, Y und Z nicht gleichzeitig für Halogen stehen.

Im einzelnen seien außer den bei den Herstellungsbeispielen genannten Verbindungen die folgenden Verbindungen der allgemeinen Formel (I) genannt:

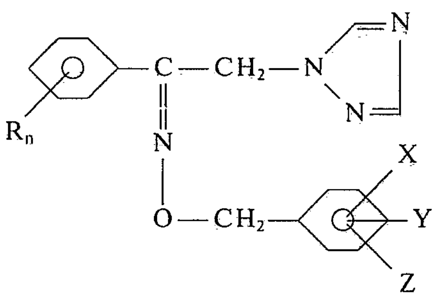

(I)

Tabelle 1

| $R_n$ | X | Y | Z |
|---|---|---|---|
| 2-Cl | 2-Cl | 3-Cl | 6-Cl |
| 2-Cl | 2-$OCH_3$ | 4-$OCH_3$ | 6-$OCH_3$ |
| 2-Cl | 2-$OCH_3$ | 3-$OCH_3$ | 4-$OCH_3$ |
| 2-Cl | 2-$OCH_3$ | 4-$OCH_3$ | 5-$OCH_3$ |
| 2-Cl | 3-$OCH_3$ | 4-$OCH_3$ | 5-$OCH_3$ |
| 2-Cl | 2-$CH_3$ | 3-$CH_3$ | 4-$OCH_3$ |
| 2-Cl | 2-$CH_3$ | 5-$CH_3$ | 4-$OCH_3$ |

Fortsetzung

| $R_n$ | X | Y | Z |
|---|---|---|---|
| 2-Cl | 2-Cl | 4,5-$O-CH_2-O-$ | |
| 2,4-$Cl_2$ | 2-$OCH_3$ | 4-$OCH_3$ | 6-$OCH_3$ |
| 2,4-$Cl_2$ | 2-$OCH_3$ | 3-$OCH_3$ | 4-$OCH_3$ |
| 2,4-$Cl_2$ | 2-$OCH_3$ | 4-$OCH_3$ | 5-$OCH_3$ |
| 2,4-$Cl_2$ | 3-$OCH_3$ | 4-$OCH_3$ | 5-$OCH_3$ |
| 2,4-$Cl_2$ | 2-$CH_3$ | 3-$CH_3$ | 4-$OCH_3$ |
| 2,4-$Cl_2$ | 2-$CH_3$ | 5-$CH_3$ | 4-$OCH_3$ |
| 2,4-$Cl_2$ | 2-Cl | 4,5-$O-CH_2-O-$ | |
| 2-$CH_3$, 4-Cl | 2-$OCH_3$ | 4-$OCH_3$ | 6-$OCH_3$ |
| 2-$CH_3$, 4-Cl | 2-$OCH_3$ | 3-$OCH_3$ | 4-$OCH_3$ |
| 2-$CH_3$, 4-Cl | 2-$OCH_3$ | 4-$OCH_3$ | 5-$OCH_3$ |
| 2-$CH_3$, 4-Cl | 3-$OCH_3$ | 4-$OCH_3$ | 5-$OCH_3$ |
| 2-$CH_3$, 4-Cl | 2-$CH_3$ | 3-$CH_3$ | 4-$CH_3$ |
| 2-$CH_3$, 4-Cl | 2-$CH_3$ | 5-$CH_3$ | 4-$OCH_3$ |
| 2-$CH_3$, 4-Cl | 2-Cl | 4,5-$O-CH_2-O-$ | |
| 2-$CH_3$, 4-Cl | 2-Cl | 4-Cl | 6-Cl |
| 2-$CH_3$, 4-Cl | 2-Cl | 3-Cl | 6-Cl |
| 2-$CH_3$, 4-Cl | 2-$CH_3$ | 4-$CH_3$ | 6-$CH_3$ |
| 2-Cl, 4-$CH_3$ | 2-$OCH_3$ | 4-$OCH_3$ | 6-$OCH_3$ |
| 2-Cl, 4-$CH_3$ | 2-$OCH_3$ | 3-$OCH_3$ | 4-$OCH_3$ |
| 2-Cl, 4-$CH_3$ | 2-$OCH_3$ | 4-$OCH_3$ | 5-$OCH_3$ |
| 2-Cl, 4-$CH_3$ | 3-$OCH_3$ | 4-$OCH_3$ | 5-$OCH_3$ |
| 2-Cl, 4-$CH_3$ | 2-$CH_3$ | 3-$CH_3$ | 4-$OCH_3$ |
| 2-Cl, 4-$CH_3$ | 2-$CH_3$ | 5-$CH_3$ | 4-$OCH_3$ |
| 2-Cl, 4-$CH_3$ | 2-Cl | 4,5-$O-CH_2-O-$ | |
| 2-Cl, 4-$CH_3$ | 2-Cl | 4-Cl | 6-Cl |
| 2-Cl, 4-$CH_3$ | 2-Cl | 3-Cl | 6-Cl |
| 2-Cl, 4-$CH_3$ | 2-$CH_3$ | 4-$CH_3$ | 6-$CH_3$ |
| 2-$CF_3$, 4-Cl | 2-$OCH_3$ | 4-$OCH_3$ | 6-$OCH_3$ |
| 2-$CF_3$, 4-Cl | 2-$OCH_3$ | 3-$OCH_3$ | 4-$OCH_3$ |
| 2-$CF_3$, 4-Cl | 2-$OCH_3$ | 4-$OCH_3$ | 5-$OCH_3$ |

Fortsetzung

| $R_n$ | X | Y | Z |
|---|---|---|---|
| 2-$CF_3$, 4-Cl | 3-$OCH_3$ | 4-$OCH_3$ | 5-$OCH_3$ |
| 2-$CF_3$, 4-Cl | 2-$CH_3$ | 3-$CH_3$ | 4-$OCH_3$ |
| 2-$CF_3$, 4-Cl | 2-$CH_3$ | 5-$CH_3$ | 4-$OCH_3$ |
| 2-$CF_3$, 4-Cl | 2-Cl | 4,5-O−$CH_2$−O− | |
| 2-$CF_3$, 4-Cl | 2-Cl | 4-Cl | 6-Cl |
| 2-$CF_3$, 4-Cl | 2-Cl | 3-Cl | 6-Cl |
| 2-$CF_3$, 4-Cl | 2-$CH_3$ | 4-$CH_3$ | 6-$CH_3$ |
| 2-$CH_3$, 4-Br | 2-$OCH_3$ | 4-$OCH_3$ | 6-$OCH_3$ |
| 2-$CH_3$, 4-Br | 2-$OCH_3$ | 3-$OCH_3$ | 4-$OCH_3$ |
| 2-$CH_3$, 4-Br | 2-$OCH_3$ | 4-$OCH_3$ | 5-$OCH_3$ |
| 2-$CH_3$, 4-Br | 3-$OCH_3$ | 4-$OCH_3$ | 5-$OCH_3$ |
| 2-$CH_3$, 4-Br | 2-$CH_3$ | 3-$CH_3$ | 4-$OCH_3$ |
| 2-$CH_3$, 4-Br | 2-$CH_3$ | 5-$CH_3$ | 4-$OCH_3$ |
| 2-$CH_3$, 4-Br | 2-Cl | 4,5-O−$CH_2$−O− | |
| 2-$CH_3$, 4-Br | 2-Cl | 4-Cl | 6-Cl |
| 2-$CH_3$, 4-Br | 2-Cl | 3-Cl | 6-Cl |
| 2-$CH_3$, 4-Br | 2-$CH_3$ | 4-$CH_3$ | 6-$CH_3$ |
| 2-Cl, 6-Cl | 2-$OCH_3$ | 4-$OCH_3$ | 6-$OCH_3$ |
| 2-Cl, 6-Cl | 2-$OCH_3$ | 3-$OCH_3$ | 4-$OCH_3$ |
| 2-Cl, 6-Cl | 2-$OCH_3$ | 4-$OCH_3$ | 5-$OCH_3$ |
| 2-Cl, 6-Cl | 3-$OCH_3$ | 4-$OCH_3$ | 5-$OCH_3$ |
| 2-Cl, 6-Cl | 2-$CH_3$ | 3-$CH_3$ | 4-$OCH_3$ |
| 2-Cl, 6-Cl | 2-$CH_3$ | 5-$CH_3$ | 4-$OCH_3$ |
| 2-Cl, 6-Cl | 2-Cl | 4,5-O−$CH_2$−O− | |
| 2-Cl, 6-Cl | 2-Cl | 4-Cl | 6-Cl |
| 2-Cl, 6-Cl | 2-Cl | 3-Cl | 6-Cl |
| 2-Cl, 6-Cl | 2-$CH_3$ | 4-$CH_3$ | 6-$CH_3$ |
| 2-$CH_3$ | 2-$OCH_3$ | 4-$OCH_3$ | 6-$OCH_3$ |
| 2-$CH_3$ | 2-$OCH_3$ | 3-$OCH_3$ | 4-$OCH_3$ |
| 2-$CH_3$ | 2-$OCH_3$ | 4-$OCH_3$ | 5-$OCH_3$ |

Fortsetzung

| $R_n$ | X | Y | Z |
|---|---|---|---|
| 2-$CH_3$ | 3-$OCH_3$ | 4-$OCH_3$ | 5-$OCH_3$ |
| 2-$CH_3$ | 2-$CH_3$ | 3-$CH_3$ | 4-$OCH_3$ |
| 2-$CH_3$ | 2-$CH_3$ | 5-$CH_3$ | 4-$OCH_3$ |
| 2-$CH_3$ | 2-Cl | 4,5-O-$CH_2$-O- | |
| 2-$CH_3$ | 2-Cl | 4-Cl | 6-Cl |
| 2-$CH_3$ | 2-Cl | 3-Cl | 6-Cl |
| 2-$CH_3$ | 2-$CH_3$ | 4-$CH_3$ | 6-$CH_3$ |

Verwendet man beispielsweise 1-(2,4-Dichlorphenyl)-1-oximino-2-(1,2,4-triazol-1-yl)-ethan und 2,4,6-Trichlorbenzylchlorid als Ausgangsstoffe, so kann der Reaktionsablauf nach der Verfahrensvariante (a) durch das folgende Formelschema wiedergegeben werden:

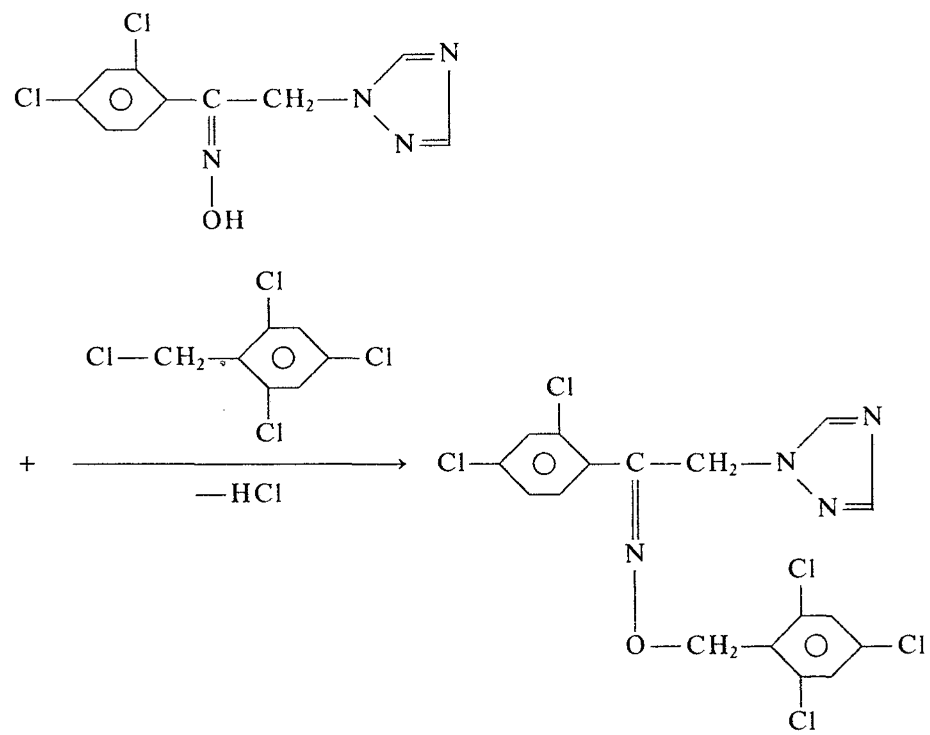


Verwendet man beispielsweise $\omega$-Chlor-2,4-dichloracetophenon-oxim-O-(2,4,6-trimethylbenzyl)-ether und 1,2,4-Triazol als Ausgangsstoffe, so kann der Reaktionsablauf nach der Verfahrensvariante (b) durch das folgende Formelschema wiedergegeben werden:

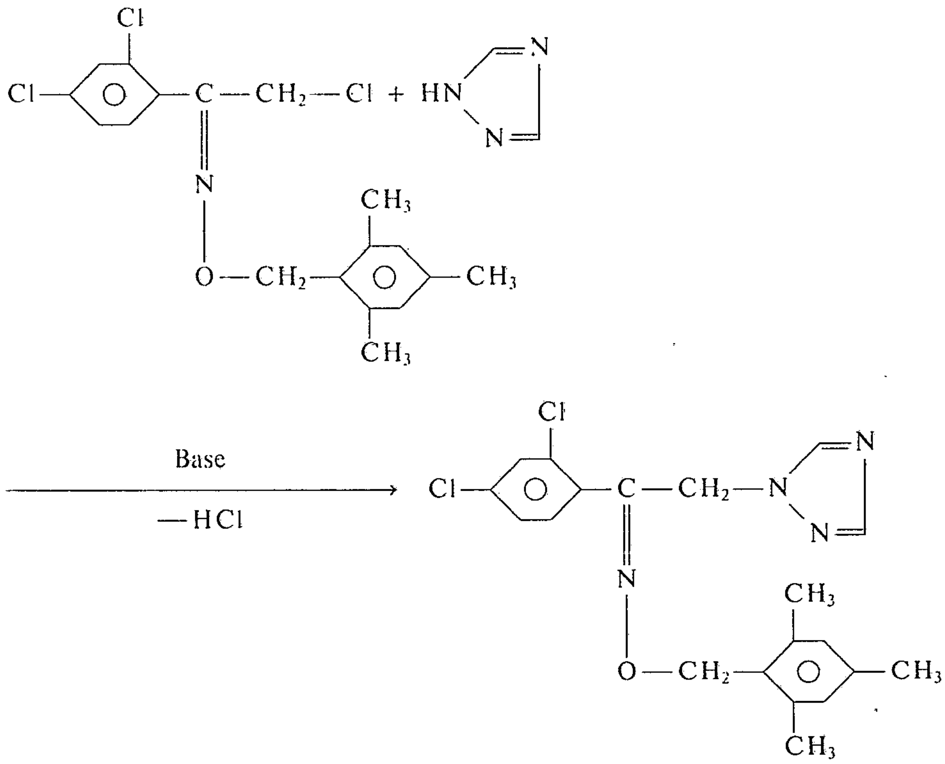


Die bei der Durchführung des erfindungsgemäßen Verfahrens (a) als Ausgangsstoffe zu verwendenden Oxime sind durch die Formel (II) allgemein definiert. In dieser Formel steht R vorzugsweise für diejenigen Reste, die bereits im Zusammenhang mit der Beschreibung der erfindungsgemäßen Stoffe der Formel (I) vorzugsweise für diesen Substituenten genannt wurden.

Die Oxime der Formel (II) sind bekannt (vgl. DE-OS 2 723 942 und DE-OS 2 657 578) und werden erhalten, indem man in einer ersten Stufe ω-Halogen-acetophenone mit 1,2,4-Triazol in Gegenwart eines inerten organischen Lösungsmittels und in Gegenwart eines Säurebinders bei Temperaturen zwischen 20 und 120°C umsetzt und die entstehenden ω-(1,2,4-Triazol-1-yl)-acetophenone in einer zweiten Stufe mit Hydroxylamin in Gegenwart eines Lösungsmittels, vorzugsweise eines Alkohols, bei 50 bis 100°C umsetzt, wobei das Hydroxylamin vorzugsweise als Hydrochlorid in Gegenwart eines Säurebindemittels eingesetzt wird.

Die außerdem für das Verfahren (a) als Ausgangsstoffe zu verwendenden Benzylhalogenide sind durch die Formel (III) allgemein definiert. In dieser Formel stehen X, Y und Z vorzugsweise für diejenigen Reste, die bereits im Zusammenhang mit der Beschreibung der erfindungsgemäßen Stoffe der Formel (I) vorzugsweise für diese Substituenten genannt wurden.

Die Benzylhalogenide der Formel (III) sind allgemein bekannte Verbindungen der organischen Chemie.

Die bei der Durchführung des erfindungsgemäßen Verfahrens (b) als Ausgangsstoffe zu verwendenden ω-Halogen-acetophenon-oximether sind durch die Formel (IV) allgemein definiert. In dieser Formel stehen R, X, Y und Z vorzugsweise für diejenigen Reste, die bereits im Zusammenhang mit der Beschreibung der erfindungsgemäßen Stoffe der Formel (I) vorzugsweise für diese Substituenten genannt wurden.

Die ω-Halogen-acetopheron-oximether der Formel (IV) sind noch nicht bekannt. Sie können jedoch nach einem neuen Verfahren erhalten werden, das Gegenstand einer vorgängigen Patentanmeldung (EP-A 00 015 456) ist.

Man erhält die Stoffe oder Formel (IV), indem man ω-Halogen-acetophenone der Formel

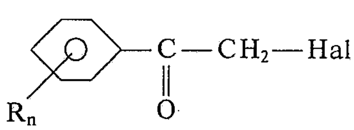
(V)

in welcher

R, n und Hal die oben angegebene Bedeutung haben,

mit Hydroxylaminen der Formel

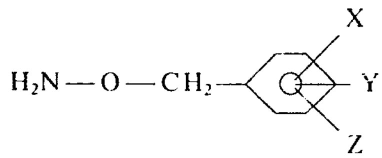

(VI)

in welcher

X, Y und Z die oben angegebene Bedeutung haben,

in Gegenwart eines Verdünnungsmittels, wie vorzugsweise eines Alkohols bzw. eines wäßrigen Alkohols, bei Temperaturen zwischen 50 und 100°C umsetzt, wobei die Hydroxylamine der Formel (VI) vorzugsweise in Form ihrer Hydrochloride in Gegenwart eines Säurebindemittels eingesetzt werden.

Zur Herstellung von physiologisch verträglichen Säureadditionssalzen der Verbindungen der Formel (I) kommen vorzugsweise folgende Säuren in Frage: Die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure, sowie Sulfonsäure, wie z. B. p-Toluolsulfonsäure und 1,5-Naphthalindisulfonsäure. Die Säureadditions-Salze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. und II. sowie IV. bis VIII. Nebengruppe in Frage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten: Halogenwasserstoffsäuren, wie z. B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z. B. durch Lösen des Metallsalzes in Alkohol, z. B. Ethanol und Hinzufügen zur Verbindung der Formel (I). Man kann Metallsalz-Komplexe in bekannter Weise, z. B. durch Abfiltrieren, Isolieren und gegebenenfalls durch Umkristallisation reinigen.

Für die erfindungsgemäße Umsetzung gemäß Verfahren (a) kommen als Verdünnungsmittel inerte organische Lösungsmittel in Frage. Hierzu gehören vorzugsweise Ether, wie Tetrahydrofuran und Dioxan; aromatische Kohlenwasserstoffe, wie Toluol und Benzol; in einzelnen Fällen auch chlorierte Kohlenwasserstoffe, wie Chloroform, Methylenchlorid oder Tetrachlorkohlenstoff; sowie Hexamethylphosphorsäure-triamid, Säureamide, wie Dimethylformamid und Sulfoxide, wie Dimethylsulfoxid.

Die erfindungsgemäße Umsetzung gemäß Verfahren (a) wird gegebenenfalls in Gegenwart einer starken Base durchgeführt. Hierzu gehören vorzugsweise Alkalimetallamide, -hydride, -hydroxide und -carbonate, wie beispielsweise Natriumamid, -carbonat, -hydroxid oder -hydrid und Kaliumamid, -carbonat, -hydroxid oder -hydrid, sowie quarternäre Ammoniumhydroxide und Phosphoniumhydroxide, wie beispielsweise Tetramethylammoniumhydroxid, Benzyltrimethyl-ammoniumhydroxid oder Dibenzyl-dimethyl-ammoniumhydroxid und Tetraphenylphosphoniumhydroxid oder Methyltriphenylphosphoniumhydroxid.

Die Reaktionstemperaturen können beim Verfahren (a) in einem größeren Bereich variiert werden. Im allgemeinen arbeitet man zwischen 20 und 150°C, vorzugsweise bei Raumtemperatur. In einzelnen Fällen ist es vorteilhaft bei der Siedetemperatur des Lösungsmittels, beispielsweise zwischen 60 und 100°C, zu arbeiten.

Bei der Durchführung des erfindungsgemäßen Verfahrens (a) setzt man auf 1 Mol Oxim der Formel (II) vorzugsweise 1 bis 3 Mol Benzylhalogenid der Formel (III) ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt, in üblicher Weise aufgearbeitet und gereinigt, gegebenenfalls wird das Salz bzw. der Metallkomplex hergestellt.

In einer bevorzugten Ausführungsform des Verfahrens (a) wird die erfindungsgemäße Umsetzung in einem Zweiphasensystem, wie beispielsweise wäßrige Natron- oder Kalilauge/Toluol oder Methylenchlorid, unter Zusatz von 0,01—1 Mol eines Phasen-Transfer-Katalysators, wie beispielsweise Ammonium- oder Phosphoniumverbindungen, durchgeführt, wobei in der organischen Phase oder an der Grenzfläche die Ethylate entstehen und mit den in der organischen Phase befindlichen Halogeniden umgesetzt werden.

Für die erfindungsgemäße Umsetzung gemäß Verfahren (b) kommen als Verdünnungsmittel vorzugsweise inerte organische Lösungsmittel in Frage. Hierzu gehören Nitrile, wie Acetonitril; Alkohole, wie Ethanol; Ether, wie Tetrahydrofuran oder Dioxan; aromatische Kohlenwasserstoffe, wie Toluol und Benzol; Formamide, wie Dimethylformamid; sowie halogenierte Kohlenwasserstoffe, wie Methylenchlorid, Tetreachlorkohlenstoff oder Chloroform.

Die erfindungsgemäße Umsetzung gemäß Verfahren (b) wird in Gegenwart eines Säurebindemittels vorgenommen. Man kann alle üblicherweise verwendbaren anorganischen oder organischen Säurebinder zugeben, wie Alkalicarbonate, z. B. Natriumcarbonat und Kaliumcarbonat; oder wie niedere tertiäre Alkylamine, Cycloalkylamine oder Aralkylamine, z. B. Triethylamin, N,N-Dimethylcyclohexylamin und N,N-Dimethylbenzylamin. Ebenfalls möglich ist ein entsprechender Überschuß an 1,2,4-Triazol.

Die Reaktionstemperaturen können beim Verfahren (b) in einem größeren Bereich variiert werden. Im allgemeinen arbeitet man zwischen 0 bis 150° C, vorzugsweise zwischen 60 bis 120° C.

Zur Durchführung des erfindungsgemäßen Verfahrens (b) setzt man auf 1 Mol der Verbindungen der Formel (IV) vorzugsweise 1 bis 2 Mol 1,2,4-Triazol und 1 bis 2 Mol Säurebinder ein. Die Isolierung der Verbindungen der Formel (I) erfolgt nach üblichen Methoden. Da die Stoffe der Formel (I) in Form von Ölen anfallen, werden sie vorzugsweise als Salze, wie insbesondere als Hydrochloride oder Nitrate, isoliert.

Gemäß einer besonderen Ausführungsform des Verfahrens (b) kann auch so vorgegangen werden, daß man zunächst die Zwischenprodukte der Formel (IV) herstellt und ohne deren Isolierung und ohne Lösungsmittelwechsel die weitere Umsetzung durchführt und die Endprodukte der Formel (I) im Rahmen eines »Eintopfverfahrens« in einem Arbeitsgang erhält.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Venturia-Arten, wie z. B. gegen den Erreger des Apfelschorfs (Fusicladium dendriticum), von Podosphaera-Arten, wie z. B. den Erreger des echten Apfelmehltaus (Podosphaera leucotricha) sowie von Getreidekrankheiten, wie Getreidemehltau und Getreiderost verwendet werden.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: Zum Beispiel natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: Zum Beispiel gebrochene und fraktionierte natürliche Gesteine wie Calcit, Marmor, Bims, Sepiolith, Dolomit sowie synthetischen Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie

Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02%, am Wirkungsort erforderlich.

In bestimmten Aufwandmengen zeigen die erfindungsgemäßen Stoffe auch eine wachstumsregulierende Wirkung.

## Herstellungsbeispiele

### Beispiel 1

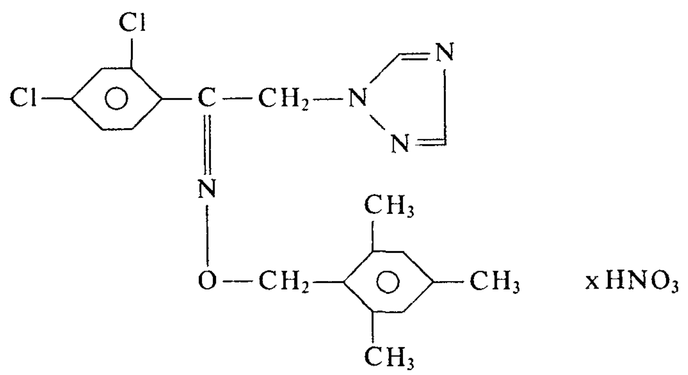

Zu 27,1 g (0,1 Mol) ω-(1,2,4-Triazol-1-yl)-oximino-2,4-dichloracetophenon und 14 g (0,1 Mol) Kaliumcarbonat in 100 ml Dimethylformamid tropft man bei 40 bis 60°C 16,8 g (0,1 Mol) 2,4,6-Trimethylbenzylchlorid in 20 ml Dimethylformamid. Man läßt 15 Stunden bei 50°C nachrühren. Danach wird die Reaktionsmischung auf 1000 ml Eiswasser gegeben. Die wäßrige Phase wird mit 500 ml Methylenchlorid und die organische Phase viermal mit je 100 ml Wasser extrahiert. Die vereinigten organischen Phasen werden über Natriumsulfat getrocknet, filtriert und durch Abdestillieren des Lösungsmittels im Vakuum eingeengt. Das resutierende Öl wird in 100 ml Aceton gelöst und 18 g 1,5-Naphthalindisulfonsäure, gelöst in 50 ml Aceton, zufiltriert. Der entstehende Niederschlag wird abgesaugt, in 200 ml Wasser/Natriumhydrogencarbonat aufgenommen und mit 200 ml Methylenchlorid ausgeschüttelt. Die organische Phase wird eingeengt und der Rückstand in 100 ml Chloroform gelöst. Unter Eiskühlung werden 3 ml konzentrierte Salpetersäure zugetropft, und der Niederschlag wird abgesaugt. Nach dem Waschen mit 50 ml Petrolether erhält man 21,6 g (46% der Theorie) 1-(2,4-Dichlorphenyl)-1-(2,4,6-trimethyl-benzylamino)-2-(1,2,4-triazol-1-yl)-ethan-nitrat vom Schmelzpunkt 74—78°C.

In entsprechender Weise und gemäß den angegebenen Verfahren werden die folgenden Verbindungen der allgemeinen Formel

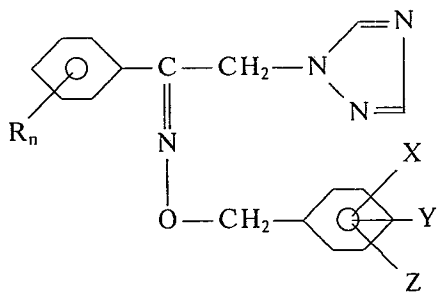

(I)

erhalten:

Tabelle 2

| Bsp. Nr. | $R_n$ | X | Y | Z | Schmelzpunkt (°C) |
|---|---|---|---|---|---|
| 2 | 2,4-$Cl_2$ | 2-Cl | 4-Cl | 6-Cl | 158–62 (x$HNO_3$) |
| 3 | 2,4-$Cl_2$ | 2-Cl | 5-Cl | 6-Cl | 153–60 (Zers.) (x$HNO_3$) |
| 4 | 2-Cl | 2-Cl | 4-Cl | 6-Cl | 153–55 (x$HNO_3$) |
| 5 | 2-Cl | 2-$CH_3$ | 4-$CH_3$ | 6-$CH_3$ | 128–30 (x$HNO_3$) |
| 6 | 2,4-$Cl_2$ | 2-$CH_3$ | 4-$CH_3$ | 6-$CH_3$ | 40 |
| 7 | 2-Cl, 4-$CH_3$ | 2-$CH_3$ | 4-$CH_3$ | 6-$CH_3$ | 100–07 |
| 8 | 2-$CF_3$, 4-Cl | 2-$CH_3$ | 4-$CH_3$ | 6-$CH_3$ | 151 (x$HNO_3$) (Zers.) |
| 9 | 2-$CH_3$, 4-Cl | 2-$CH_3$ | 4-$CH_3$ | 6-$CH_3$ | 130–34 (Zers.) (x$HNO_3$) |
| 10 | 2-$CH_3$, 4-Cl | 2-$OCH_3$ | 4-$OCH_3$ | 5-$OCH_3$ | 83 (Zers.) (x$HNO_3$) |
| 11 | 2-$CH_3$, 4-Cl | 3-$OCH_3$ | 4-$OCH_3$ | 5-$OCH_3$ | 93 (Zers.) (x$HNO_3$) |
| 12 | 2-$CH_3$, 4-Cl | 2-$OCH_3$ | 3-$OCH_3$ | 4-$OCH_3$ | 104 (Zers.) (x$HNO_3$) |
| 13 | 2-$CF_3$, 4-Cl | 2-$OCH_3$ | 4-$OCH_3$ | 5-$OCH_3$ | 80 (Zers.) (x$HNO_3$) |
| 14 | 2-$CF_3$, 4-Cl | 3-$OCH_3$ | 4-$OCH_3$ | 5-$OCH_3$ | 121 (Zers.) (x$HNO_3$) |
| 15 | 2-$CF_3$, 4-Cl | 2-$OCH_3$ | 3-$OCH_3$ | 4-$OCH_3$ | 103 (Zers.) (x$HNO_3$) |
| 16 | 2-$CH_3$, 4-Br | 3-$OCH_3$ | 4-$OCH_3$ | 5-$OCH_3$ | 114 (Zers.) (x$HNO_3$) |
| 17 | 2-$CH_3$ | 2-$OCH_3$ | 4-$OCH_3$ | 5-$OCH_3$ | 103 (Zers.) (x$HNO_3$) |
| 18 | 2-$CH_3$ | 3-$OCH_3$ | 4-$OCH_3$ | 5-$OCH_3$ | 112–17 (x$HNO_3$) |
| 19 | 2-$CH_3$, 4-Br | 2-$CH_3$ | 4-$CH_3$ | 6-$CH_3$ | 143–47 (x$HNO_3$) |
| 20 | 2-$CH_3$ | 2-$CH_3$ | 4-$CH_3$ | 6-$CH_3$ | 106–14 (x$HNO_3$) |
| 21 | 2-$CH_3$, 4-Br | 2-Cl | 4,5-O–$CH_2$–O– | | 116–18 (x$HNO_3$) |
| 22 | 2-$CF_3$, 4-Cl | 2-$CH_3$ | 4-$OCH_3$ | 5-$CH_3$ | 114–19 (x$HNO_3$) |
| 23 | 2-$CH_3$, 4-Cl | 2-Cl | 4,5-O–$CH_2$–O– | | 110–12 (Zers.) (x$HNO_3$) |
| 24 | 2-$CF_3$, 4-Cl | 2-Cl | 4,5-O–$CH_2$–O– | | 110 (Zers.) (x$HNO_3$) |
| 25 | 2,4-$Cl_2$ | 2-Cl | 4,5-O–$CH_2$–O– | | 120–24 (Zers.) (x$HNO_3$) |
| 26 | 2-Cl | 2-Cl | 4,5-O–$CH_2$–O– | | 175–78 (xHCl) |
| 27 | 2,4-$Cl_2$ | 3-$OCH_3$ | 4-$OCH_3$ | 5-$OCH_3$ | Öl |
| 28 | 2-Cl | 3-$OCH_3$ | 4-$OCH_3$ | 5-$OCH_3$ | Öl |
| 29 | 2,4-$Cl_2$ | 2-$OCH_3$ | 3-$OCH_3$ | 4-$OCH_3$ | Öl |
| 30 | 2-Cl | 2-$OCH_3$ | 3-$OCH_3$ | 4-$OCH_3$ | Öl |
| 31 | 2,4-$Cl_2$ | 2-$CH_3$ | 4-$OCH_3$ | 5-$CH_3$ | Öl |

Die gute fungizide Wirksamkeit der erfindungsgemäßen Stoffe geht aus den nachfolgenden Verwendungsbeispielen hervor.

## Beispiel A

### Fusicladium-Test (Apfel)/Protektiv

| | |
|---|---|
| Lösungsmittel: | 4,7 Gewichtsteile Aceton |
| Emulgator: | 0,3 Gewichtsteile Alkylarylpolyglykolether |
| Wasser: | 95,0 Gewichtsteile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20° C und einer relativen Luftfeuchtigkeit von 70% im Gewächshaus. Anschließend werden sie mit einer wäßrigen Konidiensuspension des Apfelschorferregers (Fusicladium dendriticum) inokuliert und 18 Stunden lang in einer Feuchtkammer bei 18 bis 20° C und 100% relativer Luftfeuchtigkeit inokuliert.

Die Pflanzen kommen dann erneut für 14 Tage ins Gewächshaus.

15 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

Wirkstoffe, Wirkstoffkonzentrationen und Ergebnisse gehen aus der nachfolgenden Tabelle hervor:

Tabelle A
Fusicladium-Test (Apfel)/protektiv

| Wirkstoff | Befall in % bei einer Wirkstoffkonzentration von 0,0025% |
|---|---|
| 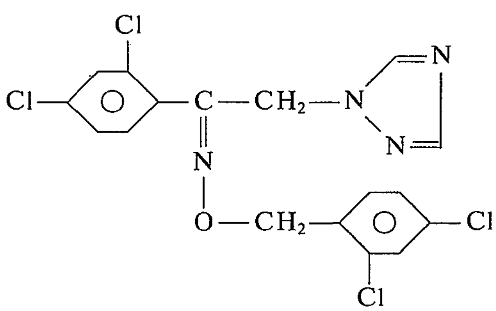 x $HNO_3$ (bekannt) | 11 |
| 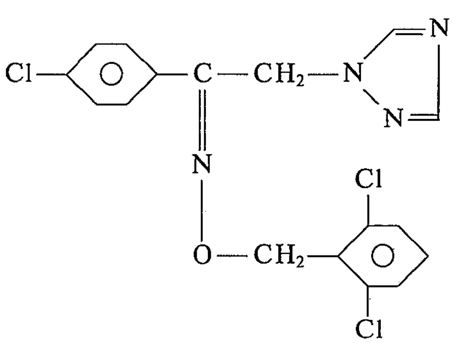 (bekannt) | 40 |

Fortsetzung

| Wirkstoff | Befall in % bei einer Wirkstoffkonzentration von 0,0025% |
|---|---|
| 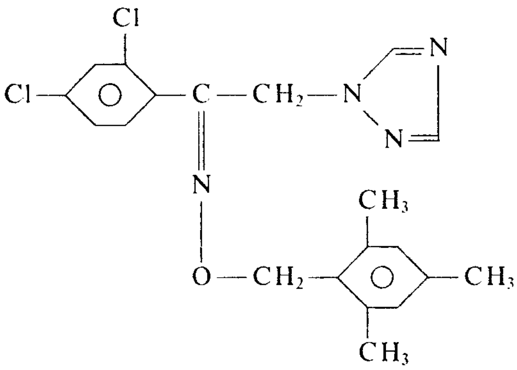 x $HNO_3$ (1) | 0 |
| 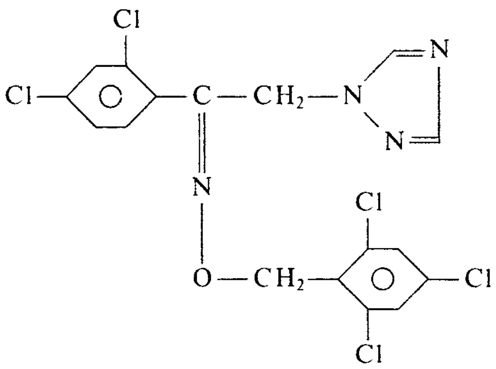 x $HNO_3$ (2) | 0 |
| 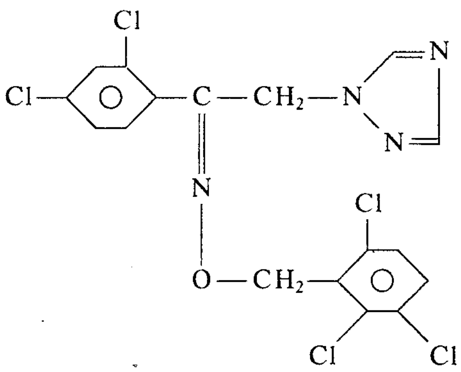 x $HNO_3$ (3) | 0 |

Beispiel B

Podosphaera-Test (Apfel)/protektiv

Lösungsmittel: 4,7 Gewichtsteile Aceton
Emulgator: 0,3 Gewichtsteile Alkyl-aryl-polyglykolether
Wasser: 95,0 Gewichtsteile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 5-Blattstadium befinden, bis zur Tropfnässe. Die Pflanzen verbleiben 24 Stunden bei 20°C und einer relativen Luftfeuchtigkeit von 70% im Gewächshaus. Anschließend werden die durch Bestäuben mit Konidien

des Apfelmehltauerregers (Podosphaera leucotricha) inokuliert und in ein Gewächshaus mit einer Temperatur von 21 bis 23° C und einer relativen Luftfeuchtigkeit von ca. 70° gebracht.

10 Tage nach der Inokulation wird der Befall der Sämlinge bestimmt. Die erhaltenen Boniturwerte werden in Prozent Befall umgerechnet. 0% bedeutet keinen Befall, 100% bedeutet, daß die Pflanzen vollständig befallen sind.

Wirkstoffe, Wirkstoffkonzentrationen und Ergebnisse gehen aus der nachfolgenden Tabelle hervor:

Tabelle B

Podosphaera-Test (Apfel)/protektiv

| Wirkstoff | Befall in % bei einer Wirkstoffkonzentration von 0,0005 % |
|---|---|
| 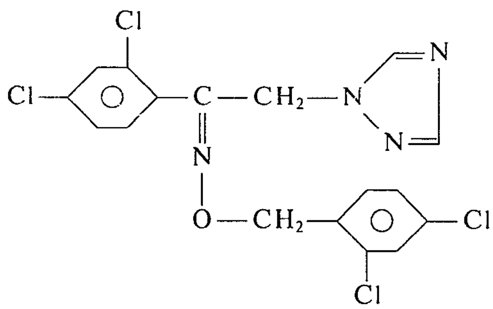 x $HNO_3$ (bekannt) | 20 |
| 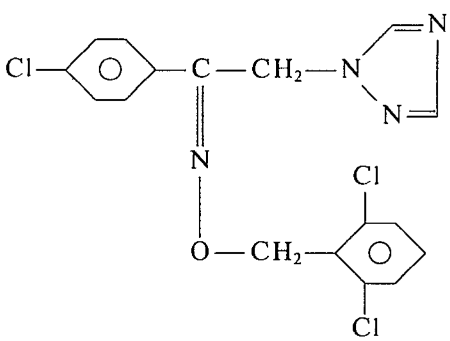 (bekannt) | 12 |
| 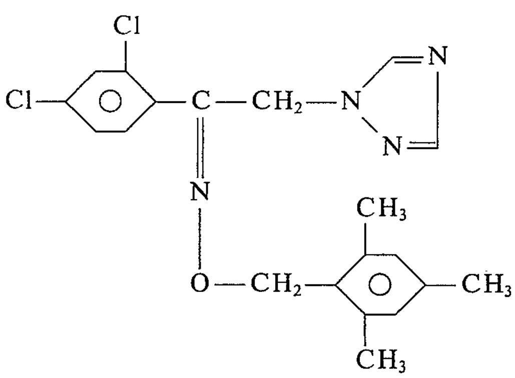 x $HNO_3$ (1) | 0 |

Fortsetzung

| Wirkstoff | Befall in % bei einer Wirkstoffkonzentration von 0,0005 % |
|---|---|
| 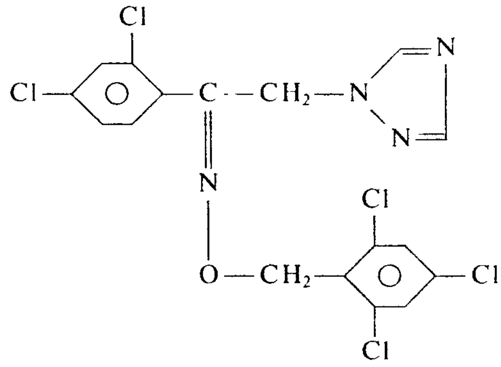 x $HNO_3$ (2) | 0 |
| 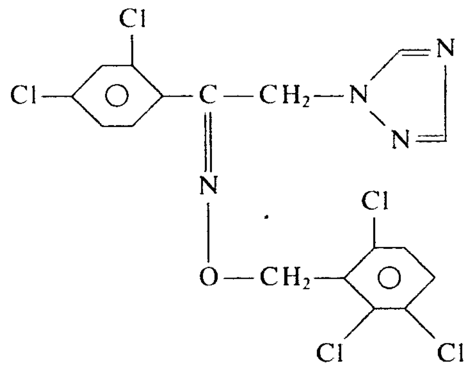 x $HNO_3$ (3) | 0 |

Beispiel C

Sproßbehandlungs-Test/Getreidemehltau/Protektiv (blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator (Alkyl-aryl-polyglykolether) auf und gibt 975 Gewichtsteile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var.hordei

Nach 6 Tagen Verweilzeit der Pflanzen bei einer Temperatur von 21 – 22° C und einer Luftfeuchtigkeit von 80 – 90% wertet man den Besatz der Pflanzen mit Mehltaupusteln aus. Der Befallsgrad wird in Prozent des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist um so wirksamer, je geringer der Mehltaubefall ist.

Wirkstoffe, Wirkstoffkonzentrationen in der Spritzbrühe und Befallsgrade gehen aus der nachfolgenden Tabelle hervor.

Tabelle C

Sproßbehandlungs-Test/Getreidemehltau/Protektiv

| Wirkstoffe | Wirkstoffkonzentration in der Spritzbrühe in Gew.-% | Befall in % der unbehandelten Kontrolle |
|---|---|---|
| 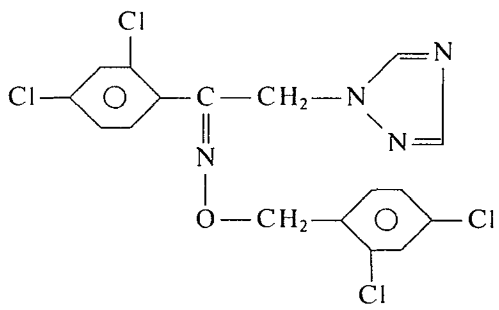 x $HNO_3$ (bekannt) | 0,0005 | 62,5 |
| 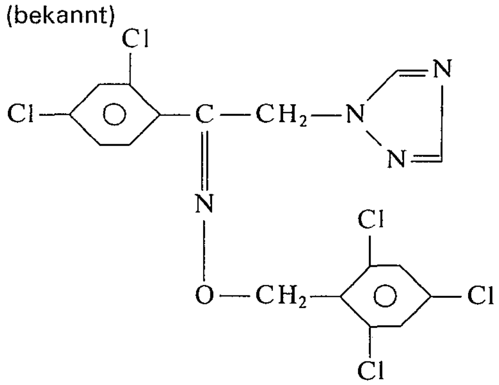 x $HNO_3$ (2) | 0,0005 | 12,5 |
| 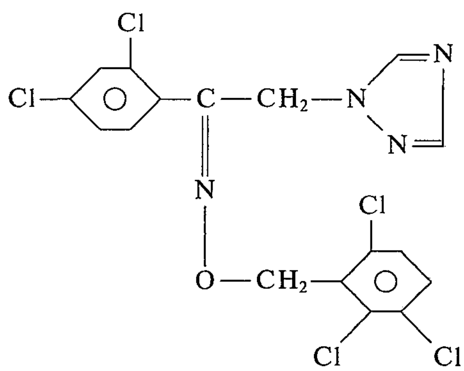 x $HNO_3$ (3) | 0,0005 | 33,8 |
| 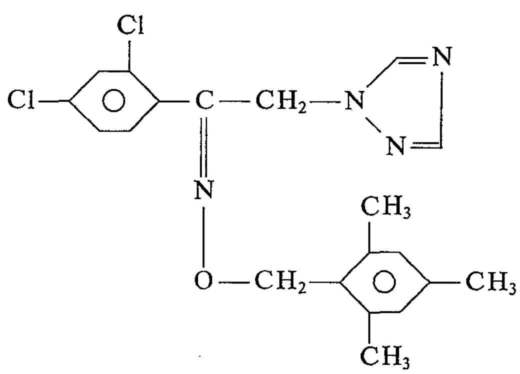 x $HNO_3$ (1) | 0,0005 | 12,5 |

Beispiel D

Sproßbehandlungs-Test/Getreiderost/protektiv (blattzerstörende Mykose)

Zur Herstellung einer zweckmäßigen Wirkstoffzubereitung nimmt man 0,25 Gewichtsteile Wirkstoff in 25 Gewichtsteilen Dimethylformamid und 0,06 Gewichtsteilen Emulgator Alkylaryl-polyglykolether auf und gibt 975 Gewichtsteile Wasser hinzu. Das Konzentrat verdünnt man mit Wasser auf die gewünschte Endkonzentration in der Spritzbrühe.

Zur Prüfung auf protektive Wirksamkeit inokuliert man einblättrige Weizenjungpflanzen der Sorte Michigan Amber mit einer Uredosporensuspension von Puccinia recondita in 0,1%igem Wasseragar. Nach Antrocknen der Sporensuspension besprüht man die Weizenpflanzen mit der Wirkstoffzubereitung taufeucht und stellt sie zur Inkubation für 24 Stunden bei etwa 20°C und einer 100%igen Luftfeuchtigkeit in ein Gewächshaus.

Nach 10 Tagen Verweilzeit der Pflanzen bei einer Temperatur von 20°C und einer Luftfeuchtigkeit von 80 – 90% wertet man den Besatz der Pflanzen mit Rostpusteln aus. Der Befallsgrad wird in Prozent des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist um so wirksamer, je geringer der Rostbefall ist.

Wirkstoffe, Wirkstoffkonzentrationen in der Spritzbrühe und Befallsgrade gehen aus der nachfolgenden Tabelle hervor.

Tabelle D

Sproßbehandlungs-Test/Getreiderost/protektiv

| Wirkstoffe | Wirkstoffkonzentration in der Spritzbrühe in Gew.-% | Befall in % der unbehandelten Kontrolle |
|---|---|---|
| 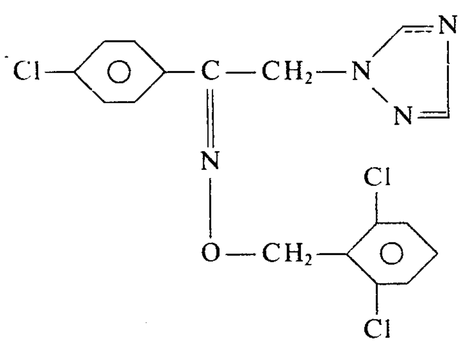 (bekannt) | 0,025 | 100 |
| 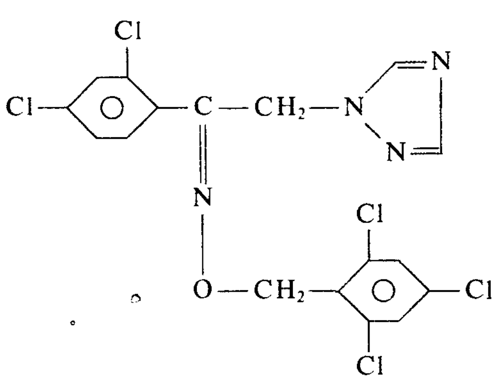 x $HNO_3$ (2) | 0,025 | 16,3 |

Fortsetzung

| Wirkstoffe | Wirkstoffkonzentration in der Spritzbrühe in Gew.-% | Befall in % der unbehandelten Kontrolle |
|---|---|---|
| 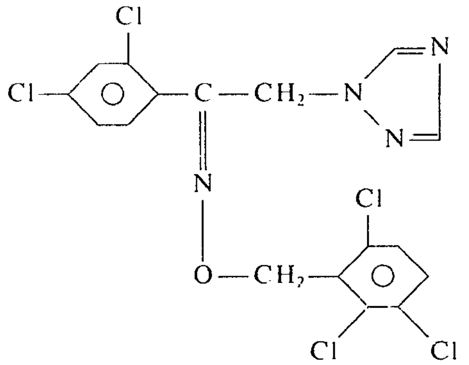 x $HNO_3$ (3) | 0,025 | 16,3 |
| 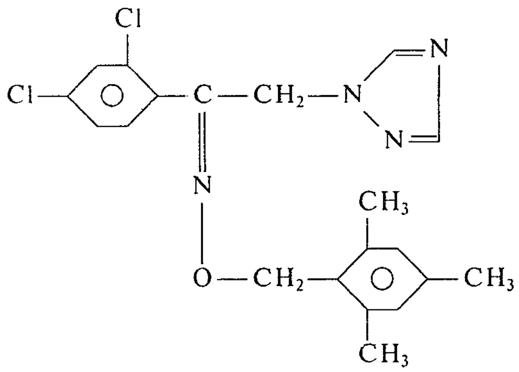 x $HNO_3$ (1) | 0,025 | 25 |

**Patentansprüche**

1. Trisubstituierte Benzyl-aximether der Formel

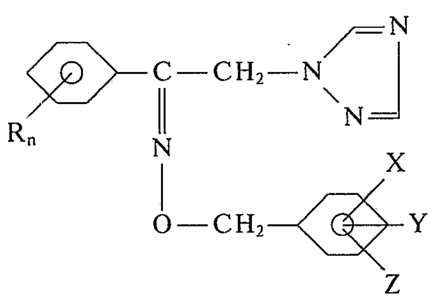

(I)

in welcher

R für Halogen, Alkyl oder Halogenalkyl steht,
n für ganze Zahlen von 1 bis 3 steht,
X, Y, Z gleich oder verschieden sind und für Alkyl, Alkoxy oder Halogen stehen, oder
Y und Z gemeinsam für Methylendioxy stehen,

und deren physiologisch verträgliche Säureadditions-Salze unbd Metallsalz-Komplexe.

2. Trisubstituierte Benzyl-oximether der Formel (I), in welcher R für Fluor, Chlor, Brom, Alkyl mit 1 bis 4 Kohlenstoffatomen oder Halogenalkyl mit 1 oder 2 Kohlenstoffatomen und 1 bis 3 Halogenatomen steht, n für 1 oder 2 steht, X, Y und Z gleich oder verschieden sind und für Alkyl mit 1 bis 4 Kohlenstoffatomen, Alkoxy mit 1 bis 4 Kohlenstoffatomen, Fluor, Chlor oder Brom stehen oder Y und Z gemeinsam für Methylendioxy stehen, und deren physiologisch verträgliche Säureadditions-Salze und Metallsalz-Komplexe.

3. Trisubstituierte Benzyl-oximether der Formel (I), in welche R für Fluor, Chlor, Brom, Methyl oder Trifluormethyl steht, n für 1 oder 2 steht, X, Y und Z gleich oder verschieden sind und für Methyl, Methoxy, Fluor, Chlor oder Brom stehen, oder Y und Z gemeinsam für Methylendioxy stehen, und deren physiologisch verträgliche Säureadditions-Salze und Metallsalz-Komplexe.

4. Verfahren zur Herstellung von trisubstituierten Benzyl-oximethern der Formel (I)

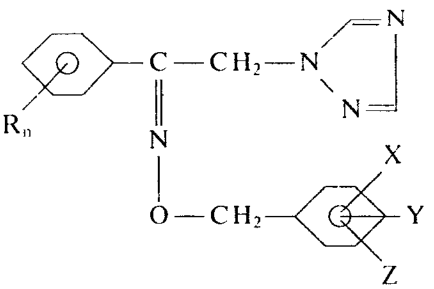

(I)

in welcher

R für Halogen, Alkyl oder Halogenalkyl steht,
n für ganze Zahlen von 1 bis 3 steht,
X, Y, Z gleich oder verschieden sind und für Alkyl, Alkoxy oder Halogen stehen, oder
Y und Z gemeinsam für Methylendioxy stehen,

sowie deren physiologisch verträglichen Säureadditions-Salzen und Metallsalzkomplexen, dadurch gekennzeichnet, daß man

a) Oxime der Formel

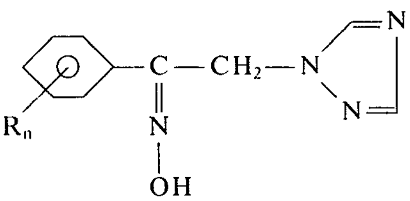

(II)

in welcher
R und n die oben angegebene Bedeutung haben,
mit Benzylhalogeniden der Formel

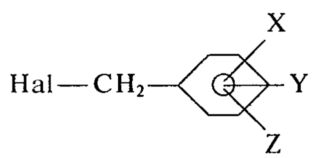

(III)

in welcher
X Y und Z die oben angegebene Bedeutung haben und
Hal für Chlor oder Brom steht,
gegebenenfalls in Gegenwart einer starken Base und in Gegenwart eines Verdünnungsmittels umsetzt, oder

b) $\omega$-Halogen-acetophenon-oximether der Formel

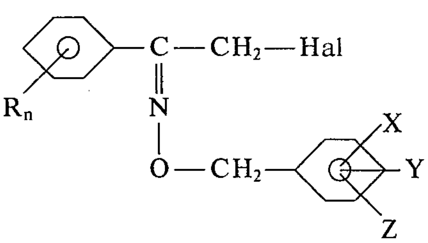

(IV)

in welcher
R, n, X, Y und Z die oben angegebene Bedeutung haben und
Hal für Chlor oder Brom steht,

mit 1,2,4-Triazol in Gegenwart eines Säurebindemittels und in Gegenwart eines Verdünnungsmittels umsetzt, und gegebenenfalls anschließend an die nach dem Verfahren (a) oder (b) erhältlichen Verbindungen eine Säure oder ein Metallsalz addiert.

5. Fungizide Mittel, gekennzeichnet durch einen Gehalt an mindestens einem trisubstituierten Benzyl-oximether der Formel (I) gemäß Anspruch 1 bzw. einem Säureadditions-Salz oder Metallsalz-Komplex eines trisubstituierten Benzyl-oximethers der Formel (I) gemäß Anspruch 1.

6. Verwendung von trisubstituierten Benzyl-oximethern der Formel (I) gemäß Anspruch 1 bzw. von Säureadditions-Salzen oder Metallsalz-Komplexen von trisubstituierten Benzyl-oximethern der Formel (I) gemäß Anspruch 1 zur Bekämpfung von Pilzen.

7. Verfahren zur Herstellung von fungiziden Mitteln, dadurch gekennzeichnet, daß man trisubstituierte Benzyl-oximether der Formel (I) gemäß Anspruch 1 bzw. Säureadditions-Salze oder Metallsalz-Komplexe von trisubstituierten Benzyl-oximethern der Formel (I) gemäß Anspruch 1 mit Streckmitteln und/oder oberflächenaktiven Mitteln vermischt.

## Claims

1. Trisubstituted benzyl oxime ethers of the formula

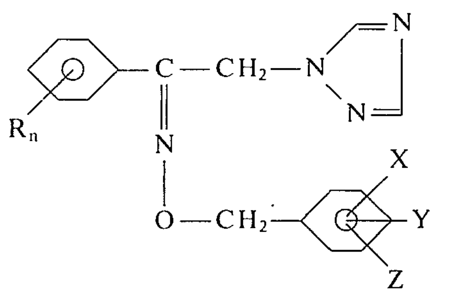

(I)

in which

| | |
|---|---|
| R | represents halogen, alkyl or halogenoalkyl, |
| n | represents an integer from 1 to 3 and |
| X, Y, Z | are identical or different and represent alkyl, alkoxy or halogen, or |
| Y and Z | together represent methylenedioxy, |

and physiologically acceptable acid addition salts and metal salt complexes thereof.

2. Trisubstituted benzyl oxime ethers of the formula (I)

in which

| | |
|---|---|
| R | represents fluorine, chlorine, bromine, alkyl with 1 to 4 carbon atoms or halogenoalkyl with 1 or 2 carbon atoms and 1 to 3 halogen atoms, |
| n | represents 1 or 2 and |
| X, Y and Z | are identical or different and represent alkyl with 1 to 4 carbon atoms, alkoxy with 1 to 4 carbon atoms, fluorine, chlorine or bromine or |
| Y and Z | together represent methylenedioxy, |

and physiologically acceptable acid addition salts and metal salt complexes thereof.

3. Trisubstituted benzyl oxime ethers of the formula (I)
in which

| | |
|---|---|
| R | represents fluorine, chlorine, bromine, methyl or trifluoromethyl, |
| n | represents 1 or 2 and |
| X, Y and Z | are identical or different and represent methyl, methoxy, fluorine, chlorine or bromine, or |
| Y and Z | together represent methylenedioxy, |

and physiologically acceptable acid addition salts and metal salt complexes thereof.

4. Process for the preparation of trisubstituted benzyl oxime ethers of the formula (I)

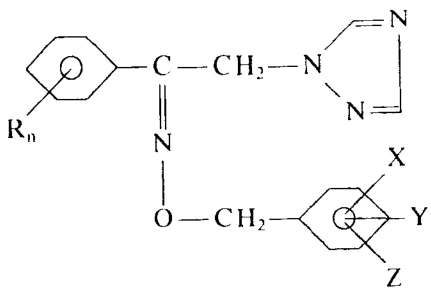

(I)

in which

R represents halogen, alkyl or halogenoalkyl,
n represents an integer from 1 to 3 and
X, Y and Z are identical or different and represent alkyl, alkoxy or halogen, or
Y and Z together represent methylenedioxy,

and of physiologically acceptable acid addition salts and metal salt complexes thereof, characterised in that

a) oximes of the formula

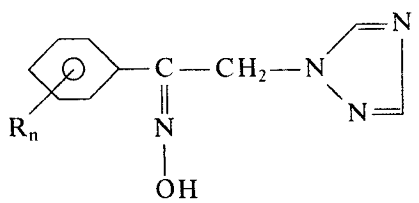

(II)

in which
R and n have the meaning indicated above,
are reacted with benzyl halides of the formula

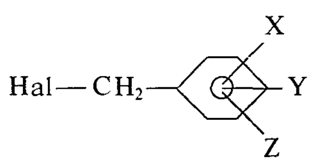

(III)

in which
X, Y and Z have the meaning indicated above and
Hal represents chlorine or bromine,
if appropriate in the presence of a strong base and in the presence of a diluent, or

b) ω-halogeno-acetophenone oxime ethers of the formula

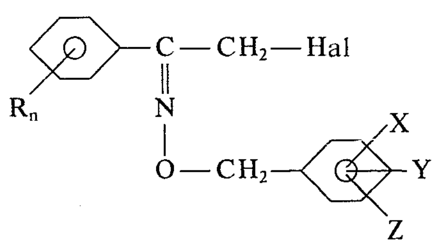

(IV)

in which
R, n, X, Y and Z have the meaning indicated above and
Hal represents chlorine or bromine,

are reacted with 1,2,4-triazole in the presence of an acid-binding agent and in the presence of a diluent, and an acid or a metal salt is then optionally added onto the compounds obtainable by process (a) or (b).

5. Fungicidal agents, characterised in that they contain at least one trisubstituted benzyl oxime ether of the formula (I) according to Claim 1 or an acid addition salt or metal salt complex of a trisubstituted benzyl oxime ether of the formula (I) according to Claim 1.

6. Use of trisubstituted benzyl oxime ethers of the formula (I) according to Claim 1 or of acid addition salts or metal salt complexes of trisubstituted benzyl oxime ethers of the formula (I) according to Claim 1, for combating fungi.

7. Process for the preparation of fungicidal agents, characterised in that trisubstituted benzyl oxime ethers of the formula (I) according to Claim 1 or acid addition salts or metal salt complexes of trisubstituted benzyl oxime ethers of the formula (I) according to Claim 1 are mixed with extenders and/or surface-active agents.

**Revendications**

1. Benzyl-oximéthers trisubstitués de formule:

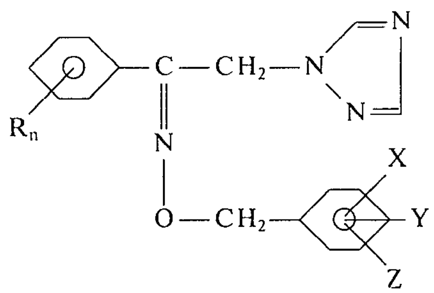

(I)

dans laquelle

| | |
|---|---|
| R | représente un atome d'halogène, un groupe alkyle ou un groupe halogénalkyle, |
| n | représente des nombres entiers de 1 à 3, |
| X, Y et Z | sont identiques ou différents et représentent chacun un groupe alkyle, un groupe alcoxy ou un atome d'halogène, ou |
| Y et Z | ensemble représentent le groupe méthylène-dioxy, |

de même que leurs complexes de sels métalliques et leurs sels d'addition d'acide physiologiquement compatibles.

2. Benzyl-oximéthers trisubstitués de formule (I) dans laquelle R représente un atome de fluor, un atome de chlore, un atome de brome, un groupe alkyle contenant 1 à 4 atomes de carbone ou un groupe halogénalkyle contenant 1 ou 2 atomes de carbone et 1 à 3 atomes d'halogènes, n représente 1 ou 2, X, Y et Z sont identiques ou différents et représentent chacun un groupe alkyle contenant 1 à 4 atomes de carbone, un groupe alcoxy contenant 1 à 4 atomes de carbone, un atome de fluor, un atome de chlore ou un atome de brome, ou encore Y et Z ensemble représentent le groupe méthylène-dioxy, de même que leurs complexes de sels métalliques et leurs sels d'additon d'acide physiologiquement compatibles.

3. Benzyl-oximéthers trisubstitués de formule (I) dans laquelle R représente un atome de fluor, un atome de chlore, un atome de brome, un groupe méthyle ou un groupe trifluorométhyle, n représente 1 ou 2, X, Y et Z sont identiques ou différents et représentent chacun un groupe méthyle, un groupe méthoxy, un atome de fluor, un atome de chlore ou un atome de brome, ou encore Y et Z ensemble représentent le groupe méthylène-dioxy,
de même que leurs complexes de sels métalliques et leurs sels d'addition d'acide physiologiquement compatibles.

4. Procédé de préparation de benzyl-oximéthers trisubstitués de formule (I):

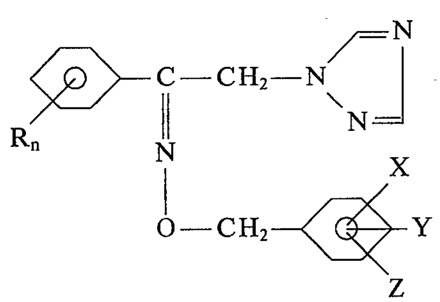

(I)

dans laquelle

| | |
|---|---|
| R | représente un atome d'halogène, un groupe alkyle ou un groupe halogénalkyle, |
| n | représente des nombres entiers de 1 à 3, |
| X, Y et Z | sont identiques ou différents et représent chacun un groupe alkyle, un groupe alcoxy ou un atome d'halogène, ou |
| Y et Z | ensemble représentent le groupe méthylène-dioxy, |

ainsi que de leurs complexes de sels métalliques et de leurs sels d'addition d'acide physiologiquement compatibles, caractérisé en ce que:

a) on fait réagir des oximes de formule:

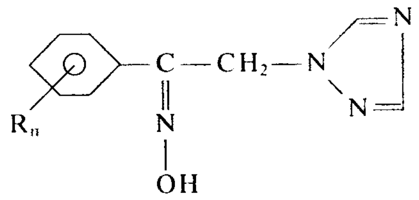

(II)

dans laquelle
R et n ont les significations indiquées ci-dessus,
avec des halogénures de benzyle de formule:

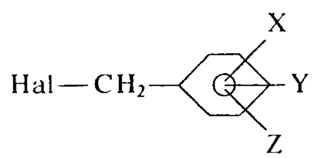

(III)

dans laquelle
X, Y et Z ont les significations indiquées ci-dessus, et
Hal représente un atome de chlore ou de brome,
éventuellement en présence d'une base forte et en présence d'un diluant, ou

b) on fait réagir des $\omega$-halogénacétophénon-oximéthers de formule:

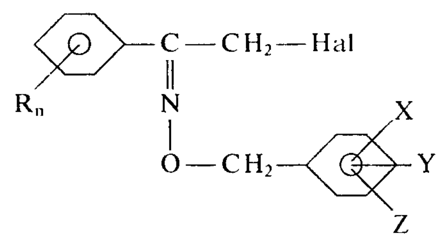

(IV)

dans laquelle
R, n, X, Y et Z ont les significations indiquées ci-dessus, et
Hal représente un atome de chlore ou de brome,

avec le 1,2,4-triazole en présence d'un agent fixateur d'acide et en présence d'un diluant, après quoi on ajoute éventuellement un acide ou un sel métallique aux composés obtenus suivant le procédé (a) ou (b).

5. Agents fongicides, caractérisés en ce qu'ils contiennent au moins un benzyl-oximéther trisubstitué de formule (I) suivant la revendication 1 ou un complexe de sels métalliques ou un sel d'addition d'acide d'un benzyl-oximéther trisubstitué de formule (I) suivant la revendication 1.

6. Utilisation de benzyl-oximéthers trisubstitués de formule (I) suivant la revendication 1 ou de complexes de sels métalliques ou de sels d'addition d'acide de benzyl-oximéthers trisubstitués de formule (I) suivant la revendication 1 puor combattre les champignons.

7. Procédé de préparation d'agents fongicides, caractérisé en ce qu'on mélange des benzyl-oximéthers trisubstitués de formule (I) suivant la revendication 1 ou des complexes de sels métalliques ou encore des sels d'additon d'acide de benzyl-oximéthers trisubstitués de formule (I) suivant la revendication 1 avec des diluants et/ou des agents tensioactifs.